# EUROPEAN PATENT APPLICATION

(11) **EP 1 142 563 A1**
(43) Date of publication of application: **10.10.2001**
(21) Application number: 99959949.1
(22) Date of filing: 21.12.1999
(51) Int. Cl.: A61K 7/13

(54) **HAIR DYE COMPOSITIONS**

(30) Priority: 22.12.1998 JP 36420098
(71) Applicant: Lion Corporation, Tokyo 130-8644 (JP)
(72) Inventor: YOSHINO, Teruhiko, Sumida-ku, Tokyo 130-8644 (JP); KIMURA, Asano, Sumida-ku, Tokyo 130-8644 (JP); ASAI, Yoshio, Sumida-ku, Tokyo 130-8644 (JP)
(74) Representative: Stuart, Ian Alexander
(86) International application number: JP9907189
(87) International publication number: WO0037032

(57) **Abstract**

A laccase-containing hair dye composition which is formulated such that the oxygen concentration therein is not more than 0.00015 wt%.

## Description

### TECHNICAL FIELD

The present invention relates to a hair dye composition containing laccase. More particularly, the present invention relates to a hair dye composition which causes less damage to hair and keeps the oxygen concentration therein low during storage, thereby improving the stability of laccase.

### BACKGROUND ART

An ordinary oxidation hair dye composition is composed of a dye precursor and an oxidizing agent, which are mixed together at the time of use and the resulting mixture is applied to the hair. It may also contain a color adjuster (so-called coupler ingredient) to impart a subtle color tone to the dyed hair.

The oxidizing agent in general use has been hydrogen peroxide. Unfortunately, hydrogen peroxide damages hair and this is a consumers' dissatisfaction with conventional oxidation hair dye compositions.

Attempts have been made to reduce hair damage by hydrogen peroxide. One of them is the replacement of hydrogen peroxide with an oxidase, such as peroxidase, laccase, and uricase, which are disclosed respectively in Japanese Patent Laid-open Nos. Sho 47-10400 and Sho 53-32132; USP No. 3251742 and Japanese Patent Laid-open No. Hei 6-172145; and Japanese Patent Laid-open No. Sho 63-246313. These disclosed technologies still have disadvantages. That is, peroxidase needs hydrogen peroxide to be added to the hair dye system because of the inherent characteristics of enzyme. Also, uricase utilizes for hair dyeing hydrogen peroxide evolved by the enzymatic reaction; therefore, it does not eliminate troubles due to hydrogen peroxide.

By contrast, laccase damages hair less than peroxidase and uricase because it does not need the hair dye system to contain hydrogen peroxide. However, laccase has the disadvantage of being unstable and becoming inactive during storage. As the result, it does not produce its effect as expected at the time of use.

There have been disclosed technologies to improve the storage stability of catalase and uricase in Japanese Patent Laid-open Nos. Hei 8-175935 and Hei 8-217652, respectively. However, there is no known technology to improve the stability of laccase. These disclosed technologies require that the composition be incorporated with a reducing agent, which inhibits the action of laccase.

### DISCLOSURE OF THE INVENTION

It is an object of the present invention to provide a laccase-containing hair dye composition which keeps laccase stable during storage and permits laccase to function effectively.

In order to achieve the above-mentioned object, the present inventors carried out extensive studies, which led to the finding that the laccase-containing hair dye composition keeps laccase active during storage if it is formulated such that the oxygen concentration therein is not more than 0.00015 wt% and it is stored in a container which prevents the oxygen concentration from increasing during storage, or if it is incorporated with an anti-oxidizing agent. The present invention is based on this finding.

It is an object of the present invention to provide a laccase-containing hair dye composition which is formulated such that the oxygen concentration therein is not more than 0.00015 wt%. It is another object of the present invention to provide a hair dye composition which is incorporated with an anti-oxidizing agent so that the oxygen concentration therein is kept low during storage.

### BEST MODE FOR CARRYING OUT THE INVENTION

A detailed description of the invention is given below. The hair dye composition according to the present invention contains laccase as an oxidizing agent. The laccase used in the present invention is an enzyme designated as E.C. 1.10.3.2. It oxidizes urushiol in the sap of lacquer tree, thereby forming Japanese lacquer. It is also ubiquitous in many vegetables and microorganisms, and it catalyzes the oxidation of aromatic compounds. The origin of laccase is not restricted in the present invention.

The hair dye composition according to the present invention may contain laccase in an amount of 0.0005 to 1%. preferably 0.005 to 0.5% (by weight), depending on the form of composition, the frequency of use, the time required for processing, and the enzyme potency. With an amount less than 0.0005%, laccase does not fully produce its effect. With an amount more than 1%, laccase does not produce any additional effect in proportion to the amount.

According to the first embodiment of the present invention, the hair dye composition containing laccase is formulated such that the oxygen concentration therein is not more than 0.00015% and the hair dye composition is stored in a container which prevents the oxygen concentration from increasing during storage. It is possible to use any known process for preparation of the composition. One way to decrease the oxygen concentration is by deaeration (to remove gas dissolved in the composition) or by aeration or filling with an inert gas such as nitrogen and argon. One way to keep the oxygen concentration low during storage is by airtightly packing the composition in a container made of a material having a low oxygen permeability. Examples of such a material include glass, aluminum, acetal resin, polyethylene terephthalate resin, and polyvinyl chloride resin. The hair dye composition according to the present invention should preferably be prepared in the form of foam incorporated with a surface active agent so that laccase produces its effect without fail. The foam should have gas therein replaced by an ordinary propellant such as carbon dioxide and LPG. The amount of the propellant in the composition should be 5-10%. The composition in the form of foam is packed into a pressure container.

According to the second embodiment of the present invention, the above-mentioned laccase containing composition is incorporated with an anti-oxidizing agent. As in the first embodiment, the composition should preferably be prepared such that the oxygen concentration therein is not more than 0.00015% and the composition should preferably be incorporated with an oxidizing agent to ensure long-term storage.

An example of the anti-oxidizing agent is ascorbic acid and a derivative thereof. Other examples include extracts from tannin-containing vegetables such as rosemary and ginkgo. Unexpectedly, these anti-oxidizing agents do not inhibit the action of laccase. The amount of the anti-oxidizing agent in the composition should be 0.01-5%, preferably 0.05-0.2%. With an amount less than 0.01%, the anti-oxidizing agent does not produce its stabilizing effect, and with an amount more than 5%, the anti-oxidizing agent would inhibit the action of laccase.

The hair dye composition according to the present invention is incorporated with a color developer, which may be selected from any compound which reacts with laccase to develop a color. Examples of such compounds include aromatic amine-based dye precursors, which are typified by p-phenylenediamine, p-aminophenol, toluene-2,5-diamine, and p-methylaminophenol sulfate. Additional examples include 3,4-diaminobenzhydrazide, 3,5-diaminobenzhydrazide, 3- hydroxytyramine, and catechin, which develop a color upon reaction with laccase. Unlike ordinary dye precursors, the above-mentioned compounds have no skin irritation on the human body and are advantageous from the safety viewpoint.

The hair dye composition according to the present invention may be incorporated with a coupler, which produces a subtle color tone which cannot be produced by the color developer used alone. The coupler is one which is commonly used for the oxidation hair dye composition; an adequate one should be selected according to the color developer used.

The hair dye composition according to the present invention may be incorporated with the color developer and coupler in an adequate amount which varies depending on the frequency of use and the form of composition. The amount is usually 0.01-10%, preferably 0.1-5%.

The hair dye composition according to the present invention may be prepared in such a way that the above-mentioned color developer is mixed with the laccase and anti-oxidizing agent. This obviates the necessity of mixing at the time of use unlike the conventional permanent hair dye composition.

The hair dye composition according to the present invention may be incorporated with a surface active agent so that it takes the form of foam. The surface active agent used for this purpose may be that of nonionic type, such as polyoxyethylene alkyl ether and fatty acid alkylolamide, or that of acylglutamic acid type. They may be used alone or in combination with one another. Polyoxyethylene alkyl ether is exemplified by polyoxyethylene stearyl and polyoxyethylene hydrogenated castor oil. Fatty acid alkylolamide is exemplified by coconut oil fatty acid diethanolamide. Surface active agents of acylglutamic acid type are exemplified by glutamic esters of C12-18 saturated or unsaturated fatty acid or a mixture thereof, such as coconut oil fatty acid, hydrogenated coconut oil fatty acid, palm oil fatty acid, hydrogenated palm oil fatty acid, tallow fatty acid, and hydrogenated tallow fatty acid. Their typical examples include N-coconut oil fatty acid acyl-L-glutamic acid triethanolamine, lauroyl-L-glutamic acid triethanolamine, sodium N-coconut oil fatty acid acyl-L-glutamate, sodium N-lauroyl-L-glutamate, sodium N-myristoyl-L-glutamate, sodium N-coconut fatty acid hydrogenated tallow fatty acid acyl-L-glutamate, and potassium N-coconut oil fatty acid acyl-L-glutamate.

The hair dye composition according to the present invention may be incorporated with a surface active agent in an amount of 0.05-5.0%, preferably 0.1-2.0%.

The hair dye composition may contain, in addition to the above-mentioned ingredients, such optional ingredients as water-soluble polymer, oil, humectant, lower alcohol, thickener, antioxidant, chelating agent, touch improver, pH adjuster, preservative, and perfume.

### EXAMPLE

In what follows, the invention will be described with reference to examples and comparative examples which are not intended to restrict the scope thereof.

### Examples 1 to 3 and Comparative Examples 1 to 3

The effect of laccase on stabilization of the hair dye composition according to the present invention was investigated in the following manner. Laccase is dissolved in HEPES buffer (pH 7.0) to give a 0.0005-0.0125 wt% laccase solution in the usual way. The solution was tested for stability after storage in two different ways.

### Storage by method A (in working examples):

The laccase solution is aerated with nitrogen gas and then stored in a glass container tightly closed with an aluminum cap.

### Storage by method B (in comparative examples):

The laccase solution is stored in a polystyrene container.

After storage at 40°C for one month, the laccase solutions were tested for laccase activity. The result is expressed in terms of residual activity compared with the initial value (100%). The activity was determined by measuring with a spectrophotometer the rate at which pigment is formed by reaction with syringaldazine as a substrate.

**Table 1**

| | Storage condition | Concentration of laccase (wt%) | Oxygen concentration in solution (wt%) | Residual activity after one month (%) |
|---|---|---|---|---|
| Example 1 | A | 0.0005 | 0.00013 | 86 |
| Example 2 | A | 0.0025 | 0.0001 | 98 |
| Example 3 | A | 0.0125 | 0.00015 | 105 |
| Comparative Example 1 | B | 0.0005 | 0.0007 | 0 |
| Comparative Example 2 | B | 0.0025 | 0.00065 | 0 |
| Comparative Example 3 | B | 0.0125 | 0.00075 | 0 |

It is noted from Table 1 that the activity of laccase is retained after storage under condition A at 40°C for one month but is lost after storage under condition B at 40°C for one month. This result indicates the effectiveness of the present invention on the stability of laccase.

### Examples 4 to 8 and Comparative examples 4 and 5

These examples demonstrate how the stability of laccase in the hair dye composition according to the present invention is affected by the oxygen concentration in the solution and the action of the anti-oxidizing agent. In each example, a 0.01 wt% laccase solution was prepared by dissolution in HEPES buffer (pH 7.0) in the usual way. The solution was aerated with nitrogen to expel oxygen. The concentration of residual oxygen in the solution was adjusted by varying the amount of nitrogen for aeration.

The thus prepared solution was stored in a tightly closed glass container at 40°C for one month, and the activity of laccase was determined. The result is expressed in terms of residual activity compared with the initial value (100%).

**Table 2**

| | Additive | Concentration of additive (wt%) | Concentration of oxygen in solution (wt%) | Residual activity after one month (%) |
|---|---|---|---|---|
| Example 4 | none | - | 0.00015 | 98 |
| Comparative Example 4 | none | - | 0.0002 | 54 |
| Comparative Example 5 | none | - | 0.0005 | 0 |
| Example 5 | L-ascorbic acid | 0.1 | 0.0002 | 103 |
| Example 6 | Rosemary extract | 0.15 | 0.00025 | 95 |
| Example 7 | Ginkgo extract | 0.075 | 0.0003 | 98 |
| Example 8 | Epicatechin polymer | 0.05 | 0.00023 | 105 |

It is noted from Table 2 that the activity of laccase decreases when the oxygen concentration in the solution exceeds 0.00015 wt% but remains stable if any of the anti-oxidizing agents shown in Table 2 is added for the same oxygen concentration.

### Examples 9 to 11

These examples demonstrate how the effect of laccase lasts while a hair dye composition containing a color developer is stored. Three hair dye compositions of the following formulations were prepared, and they were examined for the dyeing ability after storage.

| Formulation 1 (foam) for Example 9 | |
|---|---|
| p-aminophenol | 1.0 wt% |
| Toluene-2,5-diamine | 2.0 |
| Laccase | 0.05 |
| N-coconut oil fatty acid acyl-L-glutamic acid triethanolamine | 1.0 |
| Linoleic acid | 0.05 |
| Ethyl alcohol | 5.0 |
| HEPES buffer (0.1 M, pH 7) | 5.0 |
| Purified water | Balance |
| Total | 100.0 |

The above-mentioned composition (excluding laccase) was aerated with argon so as to reduce the oxygen concentration in the composition to 0.0001 wt%. Then, the composition was incorporated with as much laccase as specified above. The thus obtained composition was enclosed in a glass pressure-proof container tightly closed with an aluminum cap. The container was given LPG in an amount of 10 wt% of the composition. Thus there was obtained the gas- filled composition in the form of foam.

| Formulation 2 (cream) for Example 10 | |
|---|---|
| p-aminophenol | 0.5 wt% |
| Laccase | 0.01 |
| Liquid paraffin | 15.0 |
| Vaseline | 15.0 |
| Polyoxyethylene nonyl ethyl ether | 5.0 |
| Glycerin | 5.0 |
| L-ascorbic acid | 0.05 |
| Phosphoric acid buffer (0.1 M, pH 7) | 5.0 |
| Purified water | Balance |
| Total | 100.0 |

The above-mentioned composition (excluding laccase) was aerated with argon so as to reduce the oxygen concentration in the composition to 0.0001 wt%. Then, the composition was incorporated with as much laccase as specified above. The thus obtained composition was tightly enclosed in an aluminum cream container.

| Formulation 3 (gel) for Example 11 | |
|---|---|
| p-aminophenol | 0.5 wt% |
| Toluene-2,5-diamine | 1.0 |
| Laccase | 0.1 |
| Hydroxyethylcellulose | 1.0 |
| Polyvinylpyrrolidone | 2.0 |
| Phosphoric acid buffer (0.1 M, pH 6) | 5.0 |
| Purified water | Balance |
| Total | 100.0 |

The above-mentioned composition (excluding laccase) was aerated with nitrogen so as to reduce the oxygen concentration in the composition to 0.0001 wt%. Then, the composition was incorporated with as much laccase as specified above. The thus obtained composition was tightly enclosed in an aluminum container.

The compositions obtained in Examples 9 to 11 were stored at 40°C for one month. Each of the compositions was applied to a wisp of commercial white hair. After standing at room temperature for 30 minutes, the treated white hair was rinsed, shampooed, and air dried.

The dyed hair sample was tested for color difference (ΔE) compared with untreated white hair, by using a chroma meter. The dyeing ability was rated according to the following criterion (i). The condition of the treated hair was rated according to the following criterion (ii). The results are shown in Table 3.

### Criterion

(i) Criterion for dyeing ability:
ⓞ : ΔE is 30-40
○ : ΔE is 20-30
Δ : ΔE is 10-20
× : ΔE is 0-10

(ii) Criterion for hair condition:
ⓞ : hair is glossy and flexible
○ : hair is glossy and slightly flexible
Δ : between ○ and ×.
× : hair is dull and wiry

**Table 3**

| | Composition | Dyeing effect after storage | Condition of treated hair |
|---|---|---|---|
| Example 9 | 1 | ⓞ | ⓞ |
| Example 10 | 2 | ○ | ⓞ |
| Example 11 | 3 | ○ | ⓞ |

It is noted from Table 3 that the hair dye composition according to the present invention produces the dyeing effect even after storage for one month at 40°C, without causing damage to the hair.

### Example 12

A hair dye composition in the form of mousse was prepared according to the following formulation. The composition was deaerated so as to reduce the oxygen concentration to 0.0001 wt% and then tightly enclosed in a glass pressure-proof container with an aluminum cap. The gas in the container was replaced by LPG (in an amount such that the ratio of stock solution to LPG is 9:1). The resulting product was stored at 40°C for one month and then evaluated. It produced a good effect of uniform dyeing in brown color.

| | |
|---|---|
| p-aminophenol sulfate | 1.0 wt% |
| p-, o-cresol | 0.03 |
| Laccase | 0.3 |
| Methyl polysiloxane (Mw = 100,000) | 4.5 |
| Coconut fatty acid diethanolamide | 0.4 |
| Rosemary extract | 0.1 |
| Ethanol | 15.0 |
| Purified water | Balance |
| (pH 8) | |
| Total | 100.0 |

### Example 13

A hair dye composition in the form of foam was prepared according to the following formulation. During formulation, the composition was aerated with nitrogen so as to adjust the oxygen concentration to 0.00015 wt%. The resulting composition was tightly enclosed in a glass pressure-proof container with an aluminum cap. The gas in the container was replaced by LPG (in an amount such that the ratio of stock solution to LPG is 9.5:0.5). The resulting product was stored at 40°C for one month and then evaluated. It produced a good effect of uniform dyeing in black color.

| | |
|---|---|
| p-phenylenediamine | 0.5 wt% |
| Laccase | 0.01 |
| Acrylic resin alkanolamine (50%) | 8.0 |
| 2-sodium N-coconut oil fatty acid acyl-L-glutamate | 0.5 |
| Polyoxyethylene stearyl ether (EO = 20) | 2.0 |
| Ethanol | 15.0 |
| Glycerin | 10.0 |
| Purified water | Balance |
| (pH 7) | |
| Total | 100.0 |

### EXPLOITATION IN INDUSTRY

The hair dye composition according to the present invention retains the activity of laccase until the time of use and permits good hair dyeing by utilizing the effect of laccase.

## Claims

1. A laccase-containing hair dye composition which is formulated such that the oxygen concentration therein is not more than 0.00015 wt%.

2. A laccase-containing hair dye composition which is incorporated with an anti-oxidizing agent.

3. A hair dye composition as defined in Claim 1 or 2, which is incorporated with a surface active agent and an LPG in an amount of 5-10 wt% so that it takes the form of aerosol.
